(19) Europäisches Patentamt
European Patent Office
Office européen des brevets

(11) Publication number: **0 484 537 A1**

# (12) EUROPEAN PATENT APPLICATION published in accordance with Art. 158(3) EPC

(21) Application number: **91906513.6**

(22) Date of filing: **30.03.91**

(86) International application number: **PCT/JP91/00423**

(87) International publication number: **WO 91/15465 (17.10.91 91/24)**

(51) Int. Cl.5: **C07C 233/13**, C07C 233/15, C07C 233/18, C07C 255/54, C07C 309/66, C07C 323/41, A01N 37/22, A01N 37/34, A01N 41/04, A01N 43/40, A01N 43/54

(30) Priority: **30.03.90 JP 83472/90**

(43) Date of publication of application: **13.05.92 Bulletin 92/20**

(84) Designated Contracting States: **CH DE ES FR GB IT LI**

(71) Applicant: **CENTRAL GLASS COMPANY, LIMITED**
**5253, Oaza Okiube Ube-shi**
**Yamaguchi 755(JP)**

(72) Inventor: **TAKEMATU, Tetuo**
**612, Mine-machi**
**Utunomiya-shi, Tochigi(JP)**
Inventor: **MORI, Kaoru**
**PAAKU TAUN 5-301, 13-1, GORYO-CHO**
**HIGASHI-MATSUYAMA CITY SAITAMA(JP)**
Inventor: **KOMATA, Takeo**
**11-19, Higashida-cho**
**Kawagoe-shi, Saitama(JP)**
Inventor: **KUME, Takashi**
**11-4, Fujimi-machi**
**Kawagoe-shi, Saitama(JP)**
Inventor: **SUZUKI, Kiyoshi**
**887, Hiramatu-cho**
**Utunomiya-shi, Tochigi(JP)**
Inventor: **MINEZAKI, Matue**
**2-4, Sugahara-cho**
**Kawagoe-shi, Saitama(JP)**
Inventor: **KOMORIYA, Ikumi**
**4-8-27, Arajuku-machi**
**Kawagoe-shi, Saitama(JP)**

(74) Representative: **Dipl.-Phys.Dr. Manitz**
**Dipl.-Ing.Dipl.-Wirtsch.-Ing. Finsterwald**
**Dipl.-Phys. Rotermund Dipl.-Chem.Dr. Heyn**
**B.Sc.(Phys.) Morgan**
**Seelbergstrasse 23/25**
**W-7000 Stuttgart 50(DE)**

## (54) N-ALKYNYL-2-CHLOROACETANILIDE DERIVATIVE AND HERBICIDE CONTAINING THE SAME.

(57) An N-alkynyl-2-chloroacetanilide derivative of general formula (I) and a herbicide containing the same, which has an extremely reduced chemical injury against various crops, particularly gramineous crops, and an excellent herbicidal activity against various weeds, thus being remarkably useful as a herbicide, wherein R represents halogen, hydroxyl, lower alkoxy, lower halogenoalkoxy, alkylsufonyloxy, (un)substituted phenoxy, phenylthio, or (un)substituted nitrogenous six-membered heteroaryloxy or heteroarylthio; and $R^1$ and $R^2$ each independently

EP 0 484 537 A1

Rank Xerox (UK) Business Services
(3.08/2.19/2.0)

represents lower alkyl, halogen or trifluoromethyl.

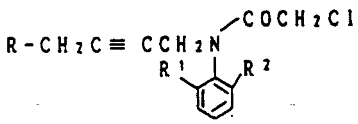

(I)

TECHNOLOGICAL FIELD

This invention relates to N-alkyne-2-chloroacetanilide derivatives which are novel compounds and herbicides containing same, and particularly herbicides which exhibit excellent activity on gramineous weeds.

BACKGROUND TECHNOLOGY

Many kinds of herbicides using chloroacetanilide compounds as the effective components are known for long, and not a few kinds of such compouds are on the market.

Examples are N-butoxymethyl-2-chloro-2',6'-diethylacetanilide (trade name: BUTACHLOR), N-propoxyethyl-2-chloro-2',6'-diethylacetanilide(trade name: PRETILACHLOR), N-methoxymethyl-2-chloro-2',6'-diethylacetanilide (trade name: ALACHLOR), etc.

These chloroacetanilide compounds exhibit excellent herbicidal action on various weeds including gramineous weeds, but it is known that these compounds are injurious to paddy and liable to suppress the growth of the above-ground part and injure the underground root part, and sometimes cause dying of paddy (missing plant).

It is an object of the present invention to solve the aforementioned problem and provide a novel compound which exhibits excellent herbicidal action but does not inflict injuries to cultivated crops, particularly to gramineous crops, even at high dosages and a herbicide which comprises the novel compound as the principal component.

DISCLOSURE OF THE INVENTION

The inventors eagerly made studies with the aim of developing a safe herbicide, which exhibits excellent herbicidal action even at a low dosage but does not injure cultivated crops, by using a chloroacetanilide compound and as a result have reached the present invention.

The present invention provides N-alkyne-2-chloroacetanilide derivatives represented by the following general formula and herbicides each containing one of the novel chloroacetanilide derivatives as the essential functional component:

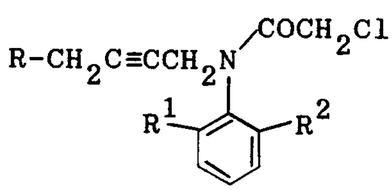

wherein R represents a halogen atom, hydroxyl group, a lower alkoxyl group, a halogenated lower alkoxyl group, an alkylsulfonyloxy group, a substituted or unsubstituted phenoxy group, phenylthio group, a substituted or unsubstituted nitrogen-containing 6-membered cyclic heteroaryloxy group or heteroarylthio group, and $R^1$ and $R^2$ each individually represent a lower alkyl group, a halogen atom or trifluoromethyl group.

A compound of the invention can be synthesized, for example, by the reaction represented by the following equation. The reaction can smoothly be carried out in a suitable solvent such as, for example, benzene, toluene, xylene, methylene chloride, chloroform, ethyl acetate, dioxane, tetrahydrofuran, diethyl ether, dimethylformamide or dimethyl sulfoxide by adding a suitable deacidifying agent such as, for example, an organic base such as triethylamine or pyridine or an inorganic base such as potassium hydroxide, sodium hydroxide or sodium hydride.

The reaction temperature is usually from -10°C to 40°C, and a preferred range is from -5°C to room temperature.

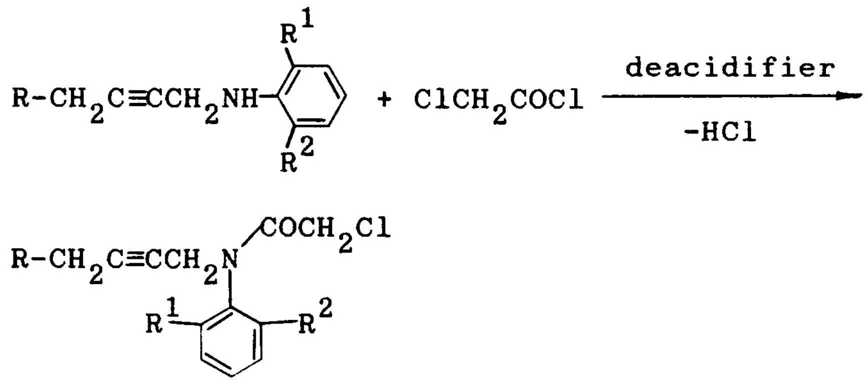


wherein R, $R^1$ and $R^2$ are as defined hereinbefore.

Hereafter the invention is described concretely with respect to examples.

EXAMPLE 1

N-Phenoxybut-2-in-2-chloro-2',6'-dimethylacetanilide (compound No. 1 in Table 1)

First 0.10 g (2.4 millimols) of sodium hydride was suspended in 10 ml of tetrahydrofuran, and a solution of 0.60 g (2.3 millimols) of N-phenoxybut-2-in-2',6'-dimethylaniline in 10 ml of tetrahydrofuran was dropped into the suspension under stirring at room temperature. After that the stirring was continued for 30 min, and then a solution of 0.26 g (2.3 millimols) of chloroacetyl chloride was added by dropping. After that stirring was continued overnight at room temperature, and then tetrahydrofuran was concentrated under reduced pressure. The residue was extracted with ethyl acetate, washed first with water, then with diluted hydrochloric acid, then with water, then with saturated aqueous solution of sodium bicarbonate and finally with water, and then dried by using anhydrous magnesium sulfate. Ethyl acetate was distilled out under reduced pressure, and the obtained crude product was refined by silica gel column chromatography thereby to obtain 0.58 g of N-phenoxybut-2-in-2-chloro-2',6'-dimethylacetanilide, which was an oil-like substance.

EXAMPLE 2

Synthesis of N-(2-pyrimidyloxybut-2-in)-2-chloro-2',6'-dimethylacetanilide (compound No. 27 in Table 1)

First 0.8 g (3.0 millimols) of N-(2-pyrimidyloxybut-2-in)-2',6'-dimethylaniline was dissolved in a mixture of 7.0 ml of benzene and 4.0 ml of ether, and then 0.52 g (3.75 millimols) of potassium carbonate was added to the solution. After adding 3 ml of water and 5 g of ice, 0.43 g (3.75 millimols) of chloroacetyl chloride was dropped into the solution at -4 to 0°C. After that stirring was continued for 30 min, and then the reaction liquid was allowed to separate into two layers. The aqueous layer was subjected to extraction with ethyl acetate, and together with the extract the organic layer was washed with water and dried. The solvent was distilled out under reduced pressure, and the obtained crude product was refined by silica gel column chromatography thereby to obtain 0.78 g of N-(2-pyrimidyloxybut-2-in)-2-chloro-2',6'-dimethylacetanilide. The melting point was 96.0-97.0°C.

EXAMPLE 3

Synthesis of N-methoxybut-2-in-2-chloro-2'6'-dimethylacetanilide (compound No. 28 in Table 1)

First 0.8 g (3.9 millimols) of N-methoxybut-2-in-2',6'-dimethylaniline was dissolved in a mixture of 5 ml of benzene and 3 ml of ether, and then 0.55 g (4.0 millimols) of potassium carbonate was added. After adding 5 ml of water and 5 g of ice, 0.49 g (4.3 millimols) of chloroacetyl chloride was dropped into the solution at -4 to 0°C. After that stirring was continued for 20 min, and then the reaction liquid was allowed to separate into two layers. The aqueous layer was subjected to extraction with ether, and together with the extract the organic layer was washed with 1 N hydrochloric acid and then with water, and dried. The solvent

was distilled out under reduced pressure, and the obtained crude product was refined by silica gel column chromatography thereby to obtain 0.88 g of N-methoxybut-2-in-2-chloro-2',6'-dimethylacetanilide, which was an oil-like substance.

EXAMPLE 4

Synthesis of N-(4-fluorophenoxybut-2-in)-2-chloro-2',6'-dimethylacetanilide (compound No. 29 in Table 1)

First 0.86 g (3.0 millimols) of N-4-fluorophenoxybut-2-in-2',6'-dimethylaniline was dissolved in a mixture of 9 ml of benzene and 3 ml of ether, and then 0.62 g (4.5 millimols) of potassium carbonate was added. After adding 3 ml of water, a solution of 0.41 g (3.6 millimols) of chloroacetyl chloride in 3 ml of ether was dropped into the initially prepared solution at -4 to 0°C. After that stirring was continued for 30 min, and then the reaction liquid was allowed to separate into two layers. The aqueous layer was subjected to extraction with ether, and together with the extract the organic layer was washed with water and then dried. The solvent was distilled out under reduced pressure, and the obtained crude product was refined by silica gel column chromatography thereby to obtain 0.74 g of N-(4-fluorophenoxybut-2-in)-2-chloro-2',6'-dimethylacetanilide. The melting point was 88.0-89.0°C.

EXAMPLE 5

Synthesis of N-methoxybut-2-in-2-chloro-2',6'-diethylacetanilide (compound No. 31 in Table 1)

First 1.50 g (6.48 millimols) of N-methoxybut-2-in-2',6'-diethylaniline was dissolved in a mixture of 8 ml of benzene and 8 ml of ether, and then 0.90 g (6.51 millimols) of potassium carbonate was added. After adding 12 ml of water and 12 g of ice, 0.81 g (7.2 millimols) of chloroacetyl chloride was dropped into the solution at -4 to 0°C. After that stirring was continued for 30 min, and then the reaction liquid was allowed to separate into two layers. The aqueous layer was subjected to extraction with ether, and together with the extract the organic layer was washed with water and then dried. The solvent was distilled out under reduced pressure, and the obtained crude product was refined by silica gel column chromatography thereby to obtain 1.82 g of N-methoxybut-2-in-2-chloro-2',6'-diethylacetanilide, which was an oil-like substance.

Table 1 shows compounds of the invention each of which was obtained by a process analogous to the processes of the foregoing examples. Among these compounds of the invention, with respect to some compounds each of which was obtained as an oil-like substance by an ordinary isolating and refining process Table 2 shows $^1$H-NMR absorption spectrum values.

The compound Nos. in Table 1 will be employed also in the following examples and experiments.

TABLE 1 (1)

| Compound No. | R | $R^1$ | $R^2$ | m.p. (°C) |
|---|---|---|---|---|
| 1 | 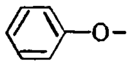 | $CH_3$ | $CH_3$ | oil-like substance |
| 2 | 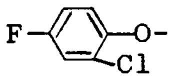 | " | " | 57.0-60.0 |
| 3 | 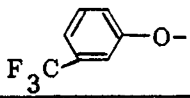 | " | " | oil-like substance |
| 4 | 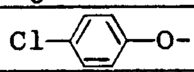 | " | " | 49.0-52.0 |
| 5 | 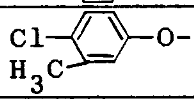 | " | " | 47.0 |
| 6 | 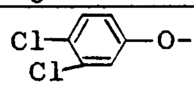 | " | " | 97.0-98.0 |
| 7 | 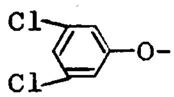 | " | " | oil-like substance |
| 8 | 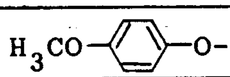 | " | " | " |
| 9 | 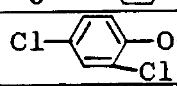 | " | " | 81.0 |
| 10 | 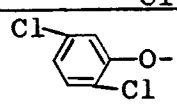 | " | " | 86.0-87.0 |
| 11 | 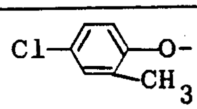 | " | " | 50.5-51.5 |
| 12 | 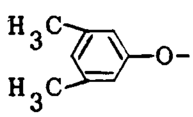 | " | " | oil-like substance |
| 13 | 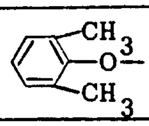 | " | " | " |
| 14 | 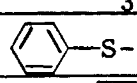 | " | " | " |
| 15 | 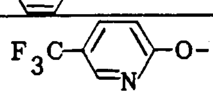 | " | " | " |

TABLE 1 (2)

| Compound No. | R | $R^1$ | $R^2$ | m.p. (°C) |
|---|---|---|---|---|
| 16 | 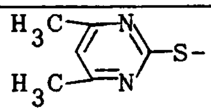 | $CH_3$ | $CH_3$ | oil-like substance |
| 17 | 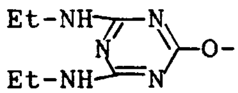 | " | " | " |
| 18 | 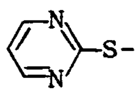 | " | " | 97.0-98.0 |
| 19 | 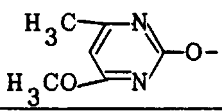 | " | " | oil-like substance |
| 20 | 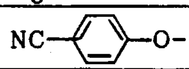 | " | " | 82.0-84.0 |
| 21 | 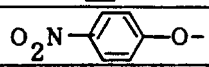 | " | " | 113.0-114.5 |
| 22 | 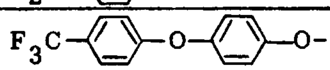 | " | " | oil-like substance |
| 23 | 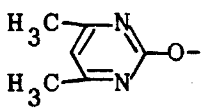 | " | " | 110.0-111.0 |
| 24 | HO- | " | " | 74.0-76.0 |
| 25 | Cl- | " | " | 63.5-64.5 |
| 26 | 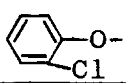 | " | " | oil-like substance |
| 27 | 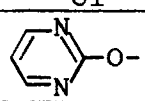 | " | " | 96.0-97.0 |
| 28 | $CH_3O-$ | " | " | oil-like substance |
| 29 | 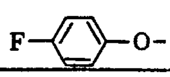 | " | " | 88.0-89.0 |
| 30 | $CH_3SO_2-$ | " | " | oil-like substance |
| 31 | $CH_3O-$ | $CH_3CH_2$ | $CH_2CH_3$ | " |
| 32 | $CH_3CH_2O-$ | $CH_3$ | $CH_3$ | " |

TABLE 1 (3)

| Compound No. | R | $R^1$ | $R^2$ | m.p. (°C) |
|---|---|---|---|---|
| 33 | 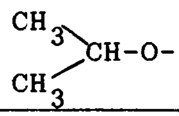 | $CH_3$ | $CH_3$ | oil-like substance |
| 34 | 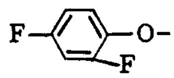 | " | " | " |
| 35 | 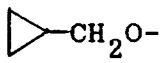 | $CH_3$ | $CH_3$ | oil-like substance |
| 36 | $CF_3CH_2O-$ | " | " | " |
| 37 | 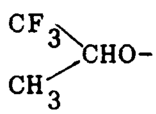 | " | " | " |
| 38 | $CH_3O-$ | " | $CH_2CH_3$ | " |
| 39 | " | $CH_3$ | 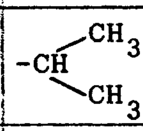 | 72.0-73.0 |
| 40 | " | " | $C(CH_3)_3$ | 54.0-54.5 |
| 41 | $CH_3O-$ | " | Cl | oil-like substance |
| 42 | " | " | $CF_3$ | 63.5-64.5 |
| 43 | " | $CH_3CH_2$ | " | oil-like substance |

TABLE 2 (1)

| Compound No. | $^1$H-NMR Absorption Spectrum Values (δ) (in $CDCl_3$) |
|---|---|
| 1 | 2.21(s, 6H), 3.67(s, 2H), 4.50(t, J = 2 Hz, 2H), 4.60(t, J = 2 Hz, 2H), 6.80-7.40(m, 8H) |
| 3 | 2.21(s, 6H), 3.67(s, 2H), 4.50(t, J = 1.5 Hz, 2H), 4.66(t, J = 1.5 Hz, 2H), 6.92-7.51(m, 7H) |
| 7 | 2.22(s, 6H), 3.37(s, 2H), 4.53(t, J = 2 Hz, 2H), 4.60(t, J = 2 Hz, 2H), 6.70-7.20(m, 6H) |
| 8 | 2.20(s, 6H), 3.67(s, 2H), 3.73(s, 3H), 4.26-4.63 (m, 4H), 6.62-6.90(m, 4H), 7.00-7.28(m, 3H) |
| 12 | 2.23(s, 6H), 2.28(s, 6H), 3.67(s, 2H), 4.51(t, J = 2 Hz, 2H), 4.55(t, J = 2 Hz, 2H), 6.57(bd, J = 11 Hz, 3H), 6.95-7.30(m, 3H) |
| 13 | 2.20(s,6H), 2.23(s, 6H), 3.67(s, 2H), 4.36(t, J = 2 Hz, 2H), 4.50(t, J = 2 Hz, 2H), 6.90-7.20 (m, 6H) |
| 14 | 2.18(s, 6H), 3.47(t, J = 1.5 Hz, 2H), 3.62(s, 2H), 4.43(t, J = 1.5 Hz, 2H), 6.95-7.44(m, 8H) |
| 15 | 2.25(s, 6H), 3.77(s, 2H), 4.53(t, J = 1.5 Hz, 2H), 4.94(s, J = 1.5 Hz, 2H), 6.82(d, J = 9 Hz, 1H), 7.00-7.20(m, 3H), 7.79(dd, J = 9.0 Hz, J = 2.0 Hz, 1H) |
| 16 | 2.25(s, 6H), 2.39(s, 6H), 3.65(s, 2H), 3.86(t, J = 1.5 Hz, 2H), 4.49(t, J = 1.5 Hz, 2H), 6.73 (s, 1H), 6.94-7.32(m, 3H) |
| 17 | 1.20(t, J = 7.0 Hz, 6H), 2.25(s, 6H), 3.18-3.61 (m, 4H), 3.68(s, 2H), 4.50(t, J = 1.5 Hz, 2H), 4.81(bt, J = 1.5 Hz, 2H), 5.42(bs, 2H), 7.00-7.38(m, 3H) |
| 19 | 2.26(s, 6H), 2.36(s, 3H), 3.70(s, 2H), 3.93(s, 3H), 4.51(t, J = 1.5 Hz, 2H), 4.90(t, J = 1.5 Hz, 2H), 6.25(s, 1H), 7.00-7.28(m, 3H) |
| 22 | 2.26(s, 6H), 3.69(s, 2H), 4.53(t, J = 2 Hz, 2H), 4.60(t, J = 2 Hz, 2H), 6.80-7.30(m, 9H), 7.57 (d, J = 8 Hz, 2H) |
| 26 | 2.20 (s, 6H), 3.67(s, 2H), 4.48(t, J = 2Hz, 2H), 4.67(t, J = 2Hz, 2H), 6.80-7.45(m, 7H) |
| 28 | 2.28(s, 6H), 3.17(s, 3H), 3.66(s, 2H), 3.95(t, J = 1.5 Hz, 2H), 4.46(t, J = 1.5 Hz, 2H), 7.1-7.5(m, 3H) |
| 30 | 2.30(s, 6H), 3.02(s, 3H), 3.71(s, 2H), 4.50(t, J = 1.5 Hz, 2H), 4.79(t, J = 1.5 Hz, 2H), 7.06-7.34(m, 3H) |

TABLE 2 (2)

| Compound No. | $^1$H-NMR Absorption Spectrum Values (δ) (in $CDCl_3$) |
|---|---|
| 31 | 1.27(t, J = 7.0 Hz, 6H), 2.59(q, J = 7.0 Hz, 4H), 3.21(s, 3H), 3.70(s, 2H), 3.99(t, J = 1.5 Hz, 2H), 4.50(t, J = 1.5 Hz, 2H), 7.1-7.5(m, 3H) |
| 32 | 1.13(t, J = 7.0 Hz, 3H), 2.30(s, 6H), 3.38(q, J = 7.0 Hz, 2H), 3.70(s, 2H), 4.02(t, J = 1.5 Hz, 2H), 4.53(t, J = 1.5 Hz, 2H), 6.9-7.4(m, 3H) |
| 33 | 1.07(d, J = 6.0 Hz, 6H), 2.29(s, 6H), 3.42-3.76 (m, J = 6.0 Hz, 1H), 3.69(s, 2H), 4.02(t, J = 1.5 Hz, 2H), 4.52(t, J = 1.5 Hz, 2H), 7.0-7.3(m, 3H) |
| 34 | 2.23(s, 6H), 3.67(s, 2H), 4.50(t, J = 2 Hz, 2H), 4.63(t, J = 2 Hz, 2H), 6.65-7.35(m, 6H) |

TABLE 2 (3)

| Compound No. | $^1$H-NMR Absorption Spectrum Values (δ) (in $CDCl_3$) |
|---|---|
| 35 | 0-0.74(m, 4H), 0.74-1.18(m, 1H), 2.31(s, 6H), 3.16 (d, J = 7 Hz, 2H), 3.71(s, 2H), 4.06(t, J = 1.5 Hz, 2H), 4.53(t, J = 1.5 Hz, 2H), 7.06-7.4(m, 3H) |
| 36 | 2.2(s, 6H), 3.65(q, J = 9 Hz, 2H), 3.71(s, 2H), 4.21(t, J = 1.5 Hz, 2H), 4.56(t, J = 1.5 Hz, 2H), 7.1-7.4(m, 3H) |
| 37 | 1.22(d, J = 7 Hz, 3H), 2.2(s, 6H), 3.7(s, 2H), 3.75(q, J = 7 Hz, 2H), 4.22(t, J = 1.5 Hz, 2H), 4.55(t, J = 1.5 Hz, 2H), 7.06-7.4(m, 3H) |
| 38 | 1.26(t, J = 8 Hz, 3H), 2.31(s, 3H), 2.62(q, J = 8 Hz, 2H), 3.2(s, 3H), 3.71(s, 2H), 3.97(t, J = 1.5 Hz, 2H), 4.52(t, J = 1.5 Hz, 2H), 7.10-7.46(m, 3H) |
| 39 | 1.22(d-d, J = 3 Hz, J = 7 Hz, 6H), 2.3(s, 3H), 2.98(m, J = 7 Hz, 1H), 3.21(s, 3H), 3.71(s, 2H), 3.96(t, J = 2 Hz, 2H), 4.5(t, J = 2 Hz, 2H), 7.03-7.4(m, 3H) |
| 40 | 1.40(s, 9H), 2.27(s, 3H), 3.08(s, 3H), 3.72, 3.76 (s, 2H), 3.89(t, J = 1.5 Hz, 2H), 4.35(d-t, J = 22 Hz, J = 1.5 Hz, 1H), 4.73(d-t, J = 22 Hz, J = 1.5 Hz, 1H), 7.06-7.54(m, 3H) |
| 41 | 2.4(s, 3H), 3.2(s, 3H), 3.72, 3.73(s, 2H), 3.96 (t, J = 1.5 Hz, 2H), 4.29(d-t, J = 21 Hz, J = 1.5 Hz, 1H), 4.86(d-t, J = 21 Hz, J = 1.5 Hz, 1H), 7.25-7.47(m, 3H) |
| 42 | 2.47(s, 3H), 3.18(s, 3H), 3.66, 3.71(s, 2H), 3.92 (t, J = 1.5 Hz, 2H), 4.18(d-t, J = 21 Hz, J = 1.5 Hz, 1H), 4.95(d-t, J = 21 Hz, J = 1.5 Hz, 1H), 7.3-7.75(m, 3H) |
| 43 | 1.31(t, J = 7 Hz, 3H), 2.75(d-q, J = 3 Hz, J = 7 Hz, 2H), 3.16 (s, 3H), 3.64, 3.69(s, 2H), 3.92(t, J = 1.5 Hz, 2H), 4.15(d-t, J = 20 Hz, J = 1.5 Hz, 1H), 4.88(d-t, J = 20 Hz, J = 1.5 Hz, 1H), 7.4-7.76(m, 3H) |

In using a compound of the invention as a herbicide, for the sake of convenience in the application of the herbicide it is possible to process the herbicide into an arbitrary form such as wettable powder, emulsion, powder, granules or flowable by using an inactive solid carrier or liquid carrier commonly used in preparing agricultural chemicals and/or an emulsifying and dispersing agent. Examples of inactive carriers are talc, clay, bentonite, kaoline, diatomaceous earth, calcium carbonate, wood flour, starch, gum arabic, water, alcohol, kerosene, benzene, xylene, n-hexane, acetone, dimethylformamide, glycol ether and N-methylpyrrolidone. Besides, it is possible to adequately incorporate auxiliary agents for formulation such as,

for example, spreader, diluent, surfactant and/or solvent. Further, it is possible to use the herbicide as a mixture with fungicide, insecticide or a still different agricultural chemical, fertilizer and/or soil conditioner with the expectation of expanded effects.

In a practical application of a compound of the invention, a suitable application dosage is somewhat variable according to related factors such as the time of application, meteorogical conditions, manner of application, form of the herbicide, location of application and weeds to be killed, but in general the application dosage (with respect to the compound of the invention) is from 0.5 to 100 g, and preferably from 3 to 50 g, per 10 ares.

The following are examples of herbicides according to the invention, though useful compounds, carriers, auxiliary agents and the proportions of the ingredients are not limited to those in these examples. In these examples the amount of each component is indicated by parts by weight.

EXAMPLE 6 (Wettable Powder)

| | |
|---|---|
| Compound No. 4 | 10 parts |
| Sodium lignin sulfonate | 1.5 parts |
| Polyoxyethylene alkylaryl ether | 1.5 parts |
| Clay | 87 parts |

These materials were mixed together until a uniform mixture was obtained, and the mixture was pulverized to obtain a wettable powder.

EXAMPLE 7 (Emulsion)

| | |
|---|---|
| Compound No. 28 | 20 parts |
| Alkylbenzenesulfonate | 5 parts |
| Polyoxyethylene alkylaryl ether | 10 parts |
| Xylene | 65 parts |

These materials were mixed until a uniform mixture was obtained, and the mixture was dissolved to obtain an emulsion.

EXAMPLE 8 (Granules)

| | |
|---|---|
| Compound No. 31 | 7 parts |
| Bentonite | 30 parts |
| Sodium alkylsulfonate | 2 parts |
| Clay | 61 parts |

These materials were mixed together and kneaded until a uniform mixture was obtained, and the mixture was granulated by an ordinary granulation method thereby to obtain granules.

The following experiments are illustrative of the herbicidal effects of the compounds of the invention.

EXPERIMENTS

Paddy soil (clay loam) was put into a pot so as to have a surface area of 1/15500 are. Uniformly mixed seeds of several kinds of weeds, viz, nobie (Echinochloa oryzicolu), broad-leaved weed, hotarui (Scripus juncoides), tamagayatsuri (Cyperus biffomis) and konagi (Monochoria vaginalis), were sown in the surface layer of the soil in each pot, and then paddy seedlings at the two- or three-leaved stage were transplanted into each pot to a depth of 2 cm, and water was fed into each pot so as to provide a 3 cm deep water layer on the soil surface. After 3 days, in other words at the initial stage of germination of nobie, a predetermined quantity of a selected compound in the form a diluted aqueous solution was dropped into the water layer in

each pot. After that the pots were kept in a glass chamber to allow paddy and the weeds to grow, and after the lapse of 4 weeks from the treatment with the selected compounds the herbicidal effects and the degree of injury to paddy were evaluated. The results are shown in Table 3. In the table the herbicidal effects and the degree of injury to paddy are indicated by numerical values, which have the following meaning.

5: completely killed
4: seriously injured
3: considerably injured
2: somewhat injured
1: slightly injured
0: not injured (growed normally)

TABLE 3 (1)

| Compound No. | Quantity of Compound g/10 a | Injury of Paddy | Herbicidal Effect | | | | |
|---|---|---|---|---|---|---|---|
| | | | nobie | broad-leaved weed | hotarui | tama-gaya-tsuri | konagi |
| 1 | 250<br>125 | 0<br>0 | 5<br>5 | 5<br>5 | 4<br>4 | 5<br>5 | 5<br>5 |
| 2 | 250<br>125 | 0<br>0 | 5<br>5 | 5<br>4 | 4<br>3 | 5<br>5 | 3<br>3 |
| 3 | 250<br>125 | 0<br>0 | 5<br>5 | 5<br>4 | 5<br>4 | 5<br>5 | 5<br>5 |
| 4 | 250<br>125 | 0<br>0 | 5<br>5 | 5<br>5 | 4<br>4 | 5<br>5 | 5<br>5 |
| 5 | 250<br>125 | 0<br>0 | 5<br>5 | 4<br>3 | 3<br>2 | 5<br>3 | 3<br>2 |
| 9 | 250<br>125 | 0<br>0 | 5<br>4 | 4<br>4 | 2<br>2 | 3<br>3 | 2<br>2 |
| 10 | 250<br>125 | 0<br>0 | 5<br>4 | 4<br>4 | 3<br>2 | 4<br>3 | 3<br>3 |
| 13 | 250<br>125 | 0<br>0 | 5<br>5 | 5<br>4 | 5<br>4 | 5<br>5 | 5<br>5 |
| 15 | 250<br>125 | 0<br>0 | 5<br>5 | 4<br>4 | 4<br>4 | 5<br>5 | 5<br>5 |
| 16 | 250<br>125 | 0<br>0 | 5<br>5 | 3<br>2 | 4<br>4 | 5<br>5 | 5<br>5 |
| 18 | 250<br>125 | 0<br>0 | 5<br>5 | 4<br>4 | 5<br>5 | 5<br>5 | 5<br>5 |
| 19 | 250<br>125 | 0<br>0 | 4<br>4 | 3<br>3 | 4<br>3 | 4<br>4 | 4<br>3 |
| 20 | 250<br>125 | 0<br>0 | 5<br>4 | 4<br>3 | 3<br>2 | 3<br>3 | 4<br>3 |
| 23 | 250<br>125 | 0<br>0 | 5<br>4 | 4<br>4 | 4<br>3 | 4<br>4 | 3<br>3 |
| 24 | 250<br>125 | 2<br>2 | 5<br>5 | 4<br>4 | 4<br>3 | 5<br>5 | 4<br>4 |

TABLE 3 (2)

| Compound No. | Quantity of Compound g/10 a | Injury of Paddy | Herbicidal Effect | | | | |
|---|---|---|---|---|---|---|---|
| | | | nobie | broad-leaved weed | hotarui | tamagayatsuri | konagi |
| 25 | 250<br>125 | 2<br>2 | 5<br>5 | 5<br>5 | 5<br>5 | 5<br>5 | 5<br>4 |
| 26 | 250<br>125 | 0<br>0 | 5<br>5 | 5<br>5 | 4<br>4 | 5<br>5 | 5<br>5 |
| 27 | 250<br>125 | 1<br>1 | 5<br>5 | 5<br>5 | 5<br>5 | 5<br>5 | 5<br>4 |
| 28 | 250<br>125 | 2<br>2 | 5<br>5 | 5<br>5 | 5<br>5 | 5<br>5 | 5<br>5 |
| 29 | 250<br>125 | 0<br>0 | 5<br>5 | 5<br>5 | 4<br>4 | 5<br>5 | 5<br>5 |
| 30 | 250<br>125 | 0<br>0 | 5<br>4 | 5<br>4 | 4<br>4 | 5<br>5 | 5<br>5 |
| 31 | 250<br>125 | 3<br>3 | 5<br>5 | 5<br>5 | 5<br>5 | 5<br>5 | 5<br>5 |
| 32 | 250<br>125 | 3<br>3 | 5<br>5 | 5<br>5 | 5<br>5 | 5<br>5 | 5<br>5 |
| 33 | 250<br>125 | 3<br>3 | 5<br>5 | 5<br>5 | 5<br>5 | 5<br>5 | 5<br>5 |
| 34 | 250<br>125 | 0<br>0 | 5<br>5 | 5<br>5 | 5<br>4 | 5<br>5 | 5<br>5 |

TABLE 3 (3)

| Compound No. | Quantity of Compound g/10 a | Injury of Paddy | Herbicidal Effect | | | | |
|---|---|---|---|---|---|---|---|
| | | | nobie | broad-leaved weed | horarui | tamagaya-tsuri | konagi |
| 35 | 200 | 3 | 5 | 4 | 5 | 5 | 5 |
| | 100 | 2 | 5 | 4 | 5 | 5 | 5 |
| 36 | 200 | 2 | 5 | 4 | 5 | 5 | 5 |
| | 100 | 1 | 5 | 4 | 5 | 5 | 5 |
| 37 | 200 | 0 | 5 | 4 | 5 | 5 | 5 |
| | 100 | 0 | 5 | 4 | 5 | 5 | 5 |
| 38 | 200 | 4 | 5 | 5 | 5 | 5 | 5 |
| | 100 | 3 | 5 | 5 | 5 | 5 | 5 |
| 39 | 200 | 3 | 5 | 5 | 5 | 5 | 5 |
| | 100 | 3 | 5 | 4 | 5 | 5 | 5 |
| 40 | 200 | 3 | 5 | 5 | 5 | 5 | 5 |
| | 100 | 3 | 5 | 4 | 5 | 5 | 5 |
| 41 | 250 | 3 | 5 | 5 | 5 | 5 | 5 |
| | 125 | 3 | 5 | 5 | 5 | 5 | 5 |
| 42 | 250 | 4 | 5 | 5 | 5 | 5 | 5 |
| | 125 | 3 | 5 | 5 | 5 | 5 | 5 |
| 43 | 250 | 4 | 5 | 5 | 5 | 5 | 5 |
| | 125 | 3 | 5 | 5 | 5 | 5 | 4.5 |

Judging comprehensively from various factors including the above experimental results, it is especially suitable to use compounmd No. 27, No. 28, No. 34, No. 35, No. 36 or No. 37 as the principal component of a herbicide according to the invention, and next to these compounds it is suitable to use compound No. 31, No. 32, No. 33, No. 38, No. 39, No. 40, No. 42 or No. 43.

MERITS OF THE INVENTION

A herbicide which contains a novel compound of the invention, an N-alkyne-2-chloro-acetanilide derivative, as the active component is a very useful herbicide since this herbicide is extremely weak in the tendency to injure various crops, and particularly gramineous crops, and is excellent in hebicidal activity to various kinds of weeds injury to various crops.

**Claims**

**1.** An N-alkyne-2-chloroacetanilide derivative represented by the following general formula,

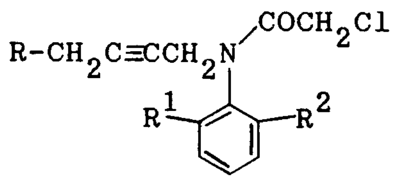

wherein R represents a halogen atom, hydroxyl group, a lower alkoxyl group, a halogenated lower alkoxyl group, an alkylsulfonyloxy group, a substituted or unsubstituted phenoxy group, phenylthio group, a substituted or unsubstituted nitrogen-containing 6-membered cyclic heteroaryloxy group or heteroarylthio group, and $R^1$ and $R^2$ each individually represent a lower alkyl group, a halogen atom or trifluoromethyl group.

2. A herbicide comprising an N-alkyne-2-chloroacetanilide derivative represented by the following formula,

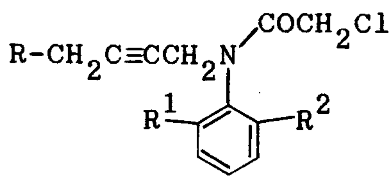

wherein R represents a halogen atom, hydroxyl group, a lower alkoxyl group, a halogenated lower alkoxyl group, an alkylsulfonyloxy group, a substituted or unsubstituted phenoxy group, phenylthio group, a substituted or unsubstituted nitrogen-containing 6-membered cyclic heteroaryloxy group or heteroarylthio group, and $R^1$ and $R^2$ each individually represent a lower alkyl group, a halogen atom or trifluoromethyl group.

3. A herbicide according to Claim 2, which is in the form of a wettable powder comprising an inactive carrier.

4. A herbicide according to Claim 2, which is in the form of an emulsion comprising an inactive carrier.

5. A herbicide according to Claim 2, which is in the form of granules comprising an inactive carrier.

6. A herbicide according to Claim 2, wherein said lower alkyl group in the general formula is methyl group or ethyl group.

7. A herbicide according to Claim 2, wherein said N-alkyne-2-chloroacetanilide derivative is N-(2-pyrimidyloxybut-2-chloro-2',6'-dimethylacetanilide.

8. A herbicide according to Claim 2, wherein said N-alkyne-2-chloroacetanilide derivative is N-methoxybut-2-in-2-chloro-2',6'-dimethylacetanilide.

9. A herbicide according to Claim 2, wherein said N-alkyne-2-chloroacetanilide derivative is N-methoxybut-2-in-2-chloro-2',6'-diethylacetanilide.

10. A herbicide according to Claim 2, wherein said N-alkyne-2-chloroacetanilide derivative is N-ethoxybut-2-in-2-chloro-2',6'-dimethylacetanilide.

11. A herbicide according to Claim 2, wherein said N-alkyne-2-chloroacetanilide derivative is N-isopropoxybut-2-in-2-chloro-2',6'-dimethylacetanilide.

12. A herbicide according to Claim 2, wherein said N-alkyne-2-chloroacetanilide derivative is N-[4-(2,4-difluorophenyl)-oxybut-2-in]-2-chloro-2',6'-dimethylacetanilide.

13. A herbicide according to Claim 2, wherein said N-alkyne-2-chloroacetanilide derivative is N-cyclopropanemethoxybut-2-in-2-chloro-2',6'-dimethylacetanilide.

14. A herbicide according to Claim 2, wherein said N-alkyne-2-chloroacetanilide derivative is N-[4-(2,2,2-trifluoroethyl)-oxybut-2-in]-2-chloro-2',6'-dimethylacetanilide.

15. A herbicide according to Claim 2, wherein said N-alkyne-2-chloroacetanilide derivative is N-[4-(1,1,1-trifluoroisopropyl)-oxybut-2-in]-2-chloro-2',6'-dimethylacetanilide.

16. A herbicide according to Claim 2, wherein said N-alkyne-2-chloroacetanilide derivative is N-methoxybut-2-in-2-chloro-2'-ethyl-6'-methylacetanilide.

17. A herbicide according to Claim 2, wherein said N-alkyne-2-chloroacetanilide derivative is N-methoxybut-2-in-2-chloro-2'-isopropyl-6'-methylacetanilide.

18. A herbicide according to Claim 2, wherein said N-alkyne-2-chloroacetanilide derivative is N-methoxybut-2-in-2-chloro-2'-methyl-6'-tert-butylacetanilide.

19. A herbicide according to Claim 2, wherein said N-alkyne-2-chloroacetanilide derivative is N-methoxybut-2-in-2-chloro-2'-methyl-6'-trifluoromethylacetanilide.

20. A herbicide according to Claim 2, wherein said N-alkyne-2-chloroacetanilide derivative is N-methoxybut-2-in-2-chloro-2'-ethyl-6'-trifluoromethylacetanilide.

# INTERNATIONAL SEARCH REPORT

International Application No PCT/JP91/00423

**I. CLASSIFICATION OF SUBJECT MATTER** (if several classification symbols apply, indicate all) [6]

According to International Patent Classification (IPC) or to both National Classification and IPC

Int. $Cl^5$ C07C233/13, C07C233/15, C07C233/18, C07C255/54, C07C309/66, C07C323/41, C07D213/64, C07D239/34,

**II. FIELDS SEARCHED**

Minimum Documentation Searched [7]

| Classification System | Classification Symbols |
|---|---|
| IPC | C07C233/13, C07C233/15, C07C233/18, C07C255/54, C07C309/66, C07C323/41, C07D213/64, C07D239/34, C07D239/38, C07D251/52, A01N37/22, A01N37/34, |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 49-094831 (Diamond Shamrock Corp.), September 9, 1974 (09. 09. 74), & NL, A, 7317271 & US, A, 400325 & GB, A, 1404916 | 1-20 |
| A | JP, A, 60-056945 (Nippon Zeon Co., Ltd.), April 2, 1985 (02. 04. 85), (Family: none) | 1-20 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 13, 1991 (13. 06. 91) | July 1, 1991 (01. 07. 91) |
| **International Searching Authority** | **Signature of Authorized Officer** |
| Japanese Patent Office | |

(Information concerning IPC to be added in the clumn I)

C07D239/38, C07D251/52, A01N37/22, A01N37/34, A01N41/04, A01N43/40, A01N43/54, A01N43/66

(Information concerning The Classification System to be added in the clumn II)

A01N41/04, A01N43/40, A01N43/54, A01N43/66